(19) Europäisches Patentamt
European Patent Office
Office européen des brevets

(11) Publication number: **0 097 352**
**B1**

(12) # EUROPEAN PATENT SPECIFICATION

(45) Date of publication of patent specification: **07.10.87**

(51) Int. Cl.$^4$: **C 07 D 417/12**

(21) Application number: **83106005.8**

(22) Date of filing: **20.06.83**

(54) **Process for the preparation of azetidine sulfonic acids.**

(30) Priority: **21.06.82 US 390728**

(43) Date of publication of application:
**04.01.84 Bulletin 84/01**

(45) Publication of the grant of the patent:
**07.10.87 Bulletin 87/41**

(84) Designated Contracting States:
**AT BE CH DE FR GB IT LI LU NL SE**

(56) References cited:
**EP-A-0 086 556**
**GB-A-2 071 650**
**US-A-4 237 128**

(73) Proprietor: **E.R. Squibb & Sons, Inc.**
**Lawrenceville-Princeton Road**
**Princeton, N.J. 08540 (US)**

(72) Inventor: **Moniot, Jerome L.**
**207C South Rd.**
**Chester, N.J. (US)**
Inventor: **Cimarusti, Christopher M.**
**278 Wargo Rd.**
**Pennington, N.J. (US)**
Inventor: **Fox, Rita T.**
**109 Farber Rd., Apt. 5-B**
**Princeton, N.J. (US)**

(74) Representative: **Vossius & Partner**
**Siebertstrasse 4 P.O. Box 86 07 67**
**D-8000 München 86 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 097 352 B1

**Description**

This invention is directed to a process for the preparation of (3S)-3-[[(2-amino-4-thiazolyl)-[(1-carboxy-1-methylethoxy)imino]acetyl]amino]-2-oxo-1 azetidinesulfonic acid and 4-substituted derivatives thereof. The process of this invention can be represented diagramatically as follows:

(I)

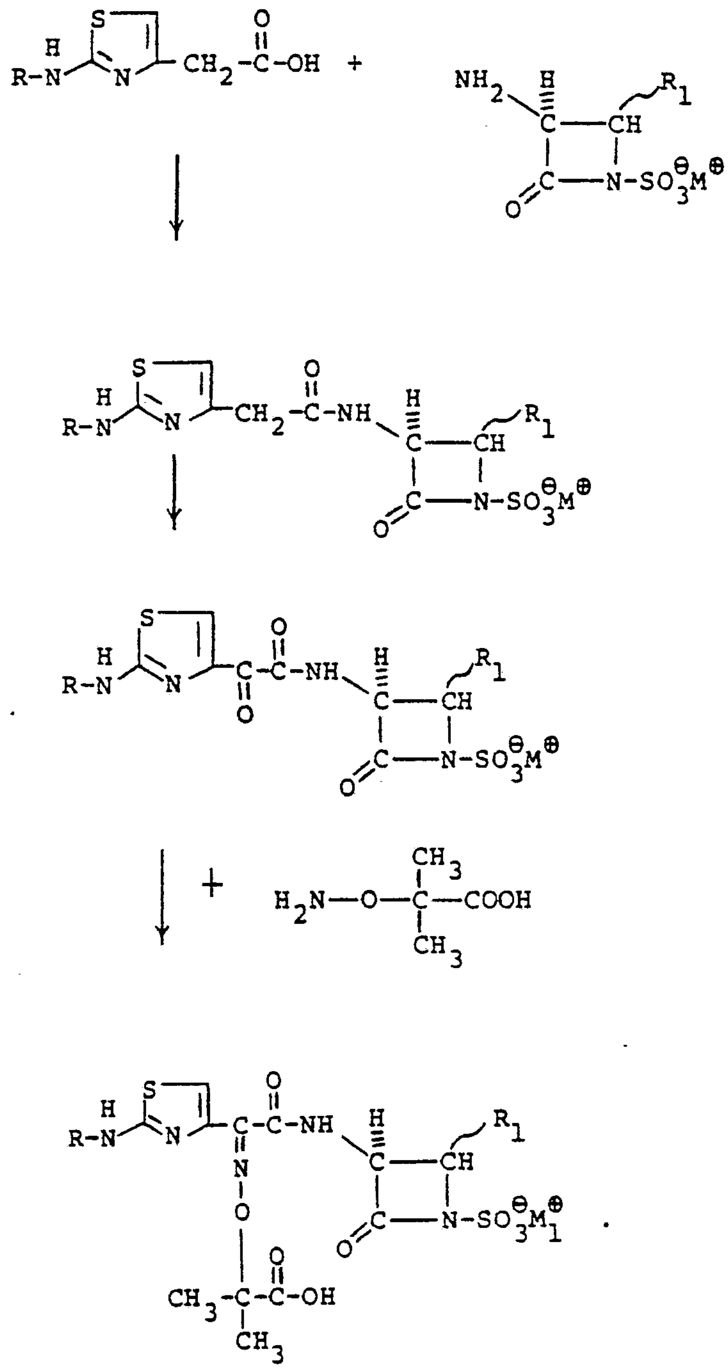

(II)

(III)

(IV)

V

In the above formulas, and throughout the specification, the symbols are as defined below.

R is hydrogen or an amino protecting group;

$R_1$ is hydrogen or $C_{1-4}$-alkyl;

$M^{\oplus}$ is an inorganic cation or a substituted ammonium ion; and

$M_1^{\oplus}$ is hydrogen, an inorganic cation, or a substituted ammonium ion.

The term "amino protecting group" refers to any group which will protect the nitrogen atom to which it is attached from reacting in the above sequence, and which, at the end of the above-described reaction

sequence, can be cleaved from the nitrogen atom under conditions that do not alter the rest of the molecule. Exemplary amino protecting groups are triphenyl-methyl, formyl, *t*-butoxycarbonyl, benzyloxycarbonyl, 1,1-dimethylpropoxycarbonyl, allyloxycarbonyl, and

$$C1(CH_2)_n-\overset{O}{\overset{\|}{C}}-$$

wherein n is 1 to 4, preferably 1 or 4.

The term "inorganic cation" refers to any positively charged inorganic atom or group of atoms. Exemplary inorganic cations ar the alkali metals *(e.g.,* lithium, sodium and potassium), the alkaline earth metals (*e.g.,* calcium and magnesium), manganic, ferrous, cobalt, thallium, managanous, and ammonium ($NH_4^{\oplus}$).

The term "substituted ammonium ion" refers to organic cations; the tri— and tetra-substituted ammonium ions are specifically contemplated. Exemplary substituted ammonium ions are the pyridinium, triethylammonium, and tetrabutylammonium salts.

The process of this invention covers intermediates which are used to prepare compounds of formula V. Those compounds of formula V wherein R is other than hydrogen can be deprotected to yield the corresponding compound of formula V wherein R is hydrogen. As described in United Kingdom patent application 2,071,650, published September 23, 1981, compounds of formula V are ß-lactam antibiotics useful for combating bacterial infections (including urinary tract infections and respiratory infections) in mammalian species, such as domesticated animals and humans. It is further disclosed that for combating bacterial infections in mammals, a compound of formula V can be administered to a mammal in need thereof in an amount of about 1.4 mg/kg/day to about 350 mg/kg/day, preferably about 14 mg/kg/day to about 100 mg/kg/day.

The reaction of an aminothiazolylacetic acid of formula I, or a salt thereof, and a (3S)-3-amino-2-oxo-1-azetidinesulfonic acid salt of formula II proceeds most readily if the aminothiazolylacetic acid of formula I is in an activated form. Activated forms of carboxylic acids are well known in the art and include acid halides, acid anhydrides (including mixed acid anhydrides), activated acid amides and activated acid esters. Mixed acid anhydrides for use in the process of this invention can be formed from an acetic acid derivative of formula I and a substituted phosphoric acid (such as dialkoxyphosphoric acid, dibenzyloxyphosphoric acid or diphenoxyphosphoric acid), a substituted phosphinic acid (such as diphenylphosphinic acid or dialkylphosphinic acid), dialkylphosphorous acid, sulfurous acid, thiosulfuric acid, sulfuric acid, a carboxylic acid such as 2,2-dimethylpropanoic acid, a carboxylic acid halide such as 2,2-dimethylpropanoyl chloride, and others. Exemplary of the activated amides which can be used in the process of this invention are those formed from a acetic acid derivative of formula I and imidazole, 4-substituted imidazoles, dimethylpyrazole, triazole, tetrazole or dimethylaminopyridine. Exemplary of the activated esters which can be used in the process of this invention are the cyanomethyl, methoxymethyl, dimethyliminomethyl, vinyl, propargyl, 4-nitrophenyl, 2,4-dinitrophenyl, trichlorophenyl, pentachlorophenyl, mesylphenyl, phenylazophenyl, phenylthio, 4-nitrophenylthio, *p*-cresylthio, carboxymethylthio, pyranyl, pyridyl, piperidyl, and 8-quinolylthio esters. Additional examples of activated esters are esters with an N-hydroxy compound such as N,N-dimethylhydroxylamine, 1-hydroxy-2(1*H*)pyridone, N-hydroxysuccinimide, N-hydroxyphthalimide, and 1-hydroxy-6-chloro-1*H*-benzotriazole.

The amides of formula III, which result from the coupling of an aminothiazolyl acetic acid of formula I (or salt thereof) and a (3S)-3-amino-2-oxo-1-azetidinesulfonic acid salt of formula II can be oxidized to yield the corresponding ketoamide of formula IV. A wide variety of oxidization procedures may be used. An exemplary procedure comprises oxidation of an amide of formula III by treatment with potassium nitrosodisulfonate in water, or a mixed aqueous system. Alternatively, oxidation can be accomplished by treatment of an amide of formula III with selenium dioxide in an inert solvent (*e.g.,* dioxane. The oxidation can also be accomplished by the use of metal catalysts in the presence of a suitable co-oxidant. Such combinations include platinum, palladium and other noble metals with air or oxygen as co-oxidant; cupric ion in solution with air or persulfate ion as co-oxidant; ferrous ion in solution with hydrogen peroxide as co-oxidant; and manganic ion, cobalt ion, thallium ion and other transition metal ions with air or oxygen gas as co-oxidant. The preferred method of oxidation of an amide of formula III comprises treatment with a solution of manganic ion in a suitable solvent, such as acetic acid, in the presence of air or oxygen as co-oxidant.

The ketoamide of formula IV can be condensed in water or in an organic solvent, with 2-aminooxy-2-methylpropanoic acid, or a salt thereof, selectively yielding the corresponding *syn*-oxime of formula V. If the pH of the condensation reaction mixture is far to the acid side *i.e.,* about 2.5 or less), the *syn*-oxime of formula V will be in the form of the zwitterion (*i.e.,* $M_1^{\oplus}$ is hydrogen). If the pH of the condensation reaction mixture is more than about 3.2, the *syn*-oxime of the formula V will be a salt corresponding to the salt of formula IV (*i.e.,* $M_1^{\oplus}$ in formula V is the same as $M^{\oplus}$ in formula IV).

The [2-(protected amino)-4-thiazolyl]acetic acid compounds of formula I are readily obtained using conventional procedures by protection of the amino group of 2-amino-4-thiazolylacetic acid; see, for example, United States patent 4,008,246. The (3S)-3-amino-2-oxo-l-azetidine-sulfonic acids of formula II are

described in the literature; see, for example, United Kingdom patent application 2,071,650, published September 23, 1981.

The following examples are specific embodiments of this invention.

Example 1

(3S-*trans*)-3-[[[2-Formylamino-4-thiazolyl]-oxoacetyl]amino]-4-methyl-2-oxo-l-azetidine-sulfonic acid, potassium salt

A) 2-Formylamino-4-thiazolylylacetic acid

In a 3-neck flask fitted with a thermometer, reflux condenser and argon inlet was placed acetic anhydride (36 ml) and formic acid 98% (16 ml) and the mixture was heated to 60°C for 90 minutes. To this solution was then added glacial acetic acid (50 ml) which caused a drop in temperature to *ca.* 40°C, and then 2-amino-4-thiazolylacetic acid (47.1 g) is added in 3 portions over 5 minutes. The temperature rose to *ca.* 60°C, was cooled to 40°C and the reaction mixture was then stirred at 40°C for 90 minutes. The solution was then cooled to 15°C, diluted with water (200 ml) and stirred at 15°C for 20 minutes. The resulting solid was removed by filtration, washed with cold water (0-5C) and dried under vacuum to give 45 g of the title compound as a powder, melting point 195-198°C.

B) (3S-*trans*)-3-[[[2-(Formylamino)-4-thiazolyl]acetyl]amino-4-methyl-2-oxo-1-azetidinesulfonic acid, potassium salt.

To a solution of pyridine (9.6 ml) and methylene chloride (150 ml) in a 1-liter flask equipped with mechanical stirring and thermometer and pre-chilled to −15°C was added a solution of pivaloyl chloride (15 ml) in methylene chloride (15 ml) at a rate to maintain a temperature below −10°C. After 2 minutes, a prechilled solution (−15°C) of 2-formylamino-4thiazolyl-acetic acid (22.5 g) and triethylamine (18.3 ml) in methylene chloride (240 ml) was added at a rate to maintain an internal temperature of below −5°C. After 5 minutes, a prechilled solution (−15°C) of (3S-*trans*)-3-amino-4-methyl-2-oxo-1-azetidinesulfonic acid (21.6 g) and triethylamine (18 ml) in acetonitrile (180 ml) was added at a rate to control internal temperature below 0°C. The reaction mixture was cooled to and maintained at −10°C for 90 minutes. The reaction was concentrated under reduced pressure to a volume of 150 ml and was then diluted with absolute ethanol to 750 ml. With mechanical stirring a 10% ethanolic potassium acetate solution (225 ml) was added. The resulting precipitate was stirred at −15°C for 20 minutes, filtered under a nitrogen atmosphere, washed with two 200 ml portions of cold absolute ethanol and dried at 40°C under vacuum to give 44.35 g of the title compound containing 1 mole of water of crystallization.

C) (3S-*trans*)-3-[[[2-(Formylamino)-4-thiazolyl]oxoacetyl]amino-4-methyl-2-oxo-1-azetidinesulfonic acid, potassium salt.

A mixture of acetic acid (50 ml), acetic anhydride (12 ml) and manganese diacetate tetrahydrate (6.32 g, 0.0258 mole) was heated to reflux (118°C) for 35 minutes under argon. The mixture was cooled to 70°C, potassium permanganate (1.03 g, 6.48 mmol) was added portionwise and the mixture was heated to reflux for 60 minutes and then cooled to 30°C. To this solution was added (3S-*trans*)-3-[[[2-(formylamino)-4-thiazolyl]acetyl]amino-4-methyl-2-oxo-1-azetidinesulfonic acid, potassium salt (5.8 g, 13 mmol) and this mixture was stirred at 30°C while a slow stream (2-10 ml/min.) of air was passed through. The reaction was monitored by tlc for completion (silica gel plates, solvent system = ethylacetate:acetonitrile:water:acetic acid, 4:4:1:1; product $R_f = 0.6$). After the thick reaction mixture was centrifuged and the solids were washed with cold glacial acetic acid (30 ml) and absolute ethanol (20 ml) and dried *in vacuo* to give a first crop of 3.55 g of the title compound.

Example 2

(3S-*trans*)-3-[[[2-(*t*-Butoxycarbonylamino)-4-thiazolyl]oxoacetyl]amino]-4-methyl-2-oxo-1-azetidinesulfonic acid, potassium salt.

A) (3S-*trans*)-3-[[[(2-*t*-Butoxycarbonylamino)-4-thiazolyl]acetyl]amino]-4-methyl-2-oxo-1-azetidinesulfonic acid, potassium salt.

To a solution of triethylamine (5.1 ml, 36 mmol) and 2-(*t*-butoxycarbonyl-amino)-4-thiazolyl-acetic acid (7.74 g, 30 mmol in methylene chloride (80 ml) at −10°C was added a solution of pivaloyl chloride (4.5 ml, 38 mmol) in methylene chloride (20 ml) over 15 minutes. To the above mixture was then added, in a steady stream over 5 minutes, a solution of (3S-*trans*)-3-amino-4-methyl-2-oxo-l-azetidinesulfonic acid (5.4 g, 30 mmol), triethylamine (4.65 ml, 33 mmol) and pyridine (2.85 ml, 36 mmol) in acetonitrile (50 ml) at −10°C; the mixture was allowed to warm to ambient temperature with stirring. After removal of the solvents *in vacuo,* the residue was dissolved in 10% aqueous tetrabutylammonium hydrogensulfate (100 ml, preadjusted to pH 3.5 with potassium bicarbonate) and extracted with methylene chloride (200 ml). The organic layer was dried over sodium sulfate, filtered and evaporated to dryness to yield 16.3 g of the title compound as the tetrabutylammonium salt.

The above salt was dissolved in acetone (75 ml), treated with a solution of potassium perfluorobutanesulfonate (8.45 g, 25 mmol) in acetone (75 ml), stirred at room temperature for 1 hour, and the solvents removed *in vacuo.* The residue was partitioned between water (75 ml) and methylene chloride-ethyl acetate (1:2, 225 ml), and the aqueous layer was lyophilized to afford 11.68 gm of the title compound.

B) (3S-*trans*)-3-[[[2-(*t*-Butoxycarbonylamino)-4-thiazolyl]oxoacetyl]amino]-4-methyl-2-oxo-1-azetidinesulfonic acid, potassium salt.

To a suspension of selenium dioxide (0.225 g) and powered 4Å molecular sieves (1.0 g) in dioxane (5.0 ml), a solution of (3S-*trans*)-3-[[[(2-*t*-butoxycarbonylamino)-4-thiazolyl]acetyl]amino]-4-methyl-2-oxo-1-azetidinesulfonic acid, tetrabutylammonium salt (1.322 g, 2 mmol) in dioxane (2.0 ml) was added. The mixture was heated to 100°C for 0.5 hours and cooled to room temperature. The cooled mixture was filtered through Celite and the filtrate was concentrated to a dense oily residue (1.65 g) which was dissolved in acetone (5.0 ml) and treated with a solution of potassium perfluorobutanesulfonate (0.5 g) in acetone (5 ml). The resulting precipitate was collected, washed with acetone and then ether and dried under vacuum to give 0.5 g of the title compound.

C) (3S-*trans*)-3-[[[2-(*t*-Butoxycarbonylamino)-4-thiazolyl]oxoacetyl]amino]-4-methyl-2-oxo-1-azetidinesulfonic acid, potassium salt (alternate oxidation).

To a filtered solution of manganic acetate dihydrate (3.48 g, 13 mmol) in glacial acetic acid (50 ml) was added (3S-*trans*)-3-[[[(2-(*t*-butoxycarbonylamino)-4-thiazolyl]acetyl]amino]-4-methyl-2-oxo-1-azetidinesulfonic acid, tetrabutylammonium salt (17.15 g, 26.5 mmol) and the mixture was stirred at 35°C under a slow air flow for 24 hours. The resulting mixture was diluted with 0.5 M monobasic potassium phosphate (75 ml) and extracted with methylene chloride (200 ml). The organic layer was concentrated *in vacuo* and taken up in ethyl acetate (150 ml), washed with water (three 50 ml portions) dried over magnesium sulfate and the solvent was removed *in vacuo*. The residue was dissolved in acetone (50 ml) and treated with a solution of potassium perfluorobutanesulfonate (8.45 g, 25 mmol) in acetone (50 ml) and was stirred at ambient temperature for one hour. The solvents were removed *in vacuo,* replaced with methylene chloride-ethyl acetate (1:2, 300 ml) and the organic solution was extracted with water (75 ml). Removal of the water under reduced pressure and drying of the residue over phosphorous pentoxide under vacuum (0.5 mm Hg) for 15 hours afforded the title compound (8.13 g).

Example 3

D) [3S-[3α(Z),4β]]-3-[[(2-Amino-4-thiazolyl)-[1-carboxy-1-methylethoxy)imino]acetyl]amino]-4-methyl-2-oxo-1-azetidinesulfonic acid

Method I

(3S - *trans*) - 3 - [[(2 - Amino - 4 - thiazolyl)oxoacetyl]amino] - 4 - methyl - 2 - oxo - 1 - azetidinesulfonic acid, potassium salt (33 mg, 0.1 mmol) was dissolved in water (0.3 ml) together with 2-aminooxy-2-methylpropanoic acid (12 mg, 0.1 mmol) and the mixture was allowed to stand for 48 hours at room temperature. Lowering the pH to 1.9 with 6N hydrochloric acid caused the product to crystallize. The product was washed with cold water followed by acetone, yielding 17 mg of product as a solid.

Method II

The procedure of Method I was repeated using 23 mg (0.1 mmol) of the trifluoroacetate salt of 2-aminooxy-2-methylpropanoic acid. The desired product precipitated from solution, and after cooling, was collected and dried.

Method III

(3S - *trans*) - 3 - [[(2 - Amino - 4 - thiazolyl)oxoacetyl]amino] - 4 - methyl - 2 - oxo - 1 - azetidinesulfonic acid, potassium salt (50 mg, 0.15 mmol) and 2-aminooxy-2-methylpropanoic acid (18 mg, 0.15 mmol) were dissolved in 0.5M pH 5.8 phosphate buffer and stirred at room temperature for 24 hours. After standing for 24 hours at 5°C, the pH was lowered to 2 with 1N hydrochloric acid and the solution was concentrated under a stream of nitrogen. After cooling at 5°C, crystals were collected from the concentrated solution, washed with cold water, then with acetone-ether, and dried to give 30 mg of product as a powder.

Method IV

(3S - *trans*) - 3 - [[(2 - Amino - 4 - thiazolyl)oxoacetyl]amino] - 4 - methyl - 2 - oxo - 1 - azetidinesulfonic acid, potassium salt (25 mg, 0.067 mmol) and 2-aminooxy-2-methylpropanoic acid (9 mg, 0.076 mmol) dissolved in water (0.3 ml) were heated to 60°C. After 2 hours the mixture was cooled, the pH was lowered to 1.8 with 1N hydrochloric acid, and after standing at 5°C the crystallized product was collected. Washing with acetone-ether and drying gave 14 mg of product as a powder.

Method V

(3S - *trans*) - 3 - [[(2 - Amino - 4 - thiazolyl)oxoacetyl]amino] - 4 - methyl - 2 - oxo - 1 - azetidinesulfonic acid, potassium salt (50 mg, 0.134 mmol) and 2-aminooxy-2-methylpropanoic acid (105 mg, 0.450 mmol) were dissolved in dimethylformamide (1 ml) and stirred at room temperature for 24 hours. Solvent was removed *in vacuo* and residue was crystallized from water on cooling. The solid was collected and washed with cold water, then with acetone-ether, and dried to give 27 mg of product as a powder.

Example 4

[3S-[3α(Z),4β]]-3-[[[2-(Formylamino)-4-thiazolyl][(1-carboxy-1-methylethoxy)imino]-acetyl]amino]-4-methyl-2-oxo-1-azetidinesulfonic acid

A solution of 2.33 g (10.00 mmole) of 2-aminooxy-2-methylpropanoic acid, trifluoroacetate salt, in 5 ml of water was made. The pH of this solution was adjusted from 0.5 to 2 with aqeuous saturated potassium bicarbonate, then brought to 10 ml total volume with water. To this solution was added a 10 ml aqueous suspension of 2.0g (5.0 mmole) of [3S-*trans*-3-[[[(formylamino)-4-thiazolyl]oxoacetyl]amino]-4-methyl-2-oxo-1-azetidinesulfonic acid, potassium salt. The suspension was stirred at ambient temperature, (ca. 20-25°C); the pH was maintained at 2 by dropwise addition of saturated aqueous potassium bicarbonate. The suspension became a nearly clear solution after 5 hours. The solution was stirred for one additional hour, then filtered, and the filtrate lyophilized overnight. Crude lyophilate was used directly in the next step. Thin layer chromatography of the crude lyophilate showed the title compound to be the major product, the non-formylated analog as a by-product, and a more polar product.

Example 5

[3S-[3α(Z),4β]]-3-[[(2-Amino-4-thiazolyl)[(1-carboxyl-1-methylethoxy)imino]-acetyl]amino]-4-methyl-2-oxo-1-azetidinesulfonic acid

Method I

A solution of 4.3 ml of 2N hydrochloric acid was made by adding 0.71 ml of concentrated hydrochloric acid (*ca.* 12N) to 3.6 ml of water. To this solution was added 1.94 g (2.15 mmole) of crude lyophilate containing [3S - [3α(Z),4β]] - 3 - [[[(2 - (formylamino) - 4 - thiazolyl][(1 - carboxy - 1 - methylethoxy)imino] - acetyl]amino] - 4 - methyl - 2 - oxo - 1 - azetidinesulfonic acid. The solution was stirred at ambient temperature and in 90 minutes a precipitate formed. Stirring was continued for an additional 6 hours, and the reaction solution was diluted withh 4 ml of isopropanol and filtered. The first filtrate was separated and the first crop was washed with 10 ml of isopropanol and 30 ml of ethyl acetate, and dried *in vacuo* at ambient temperature overnight, yielding 0.587 g of solid. Quantitative analysis versus standard showed it contained 81% of the desired product.

The first crop filtrate was diluted with a second 4 ml portion of isopropanol and stored at 0-5°C overnight. A second crop of product was collected by filtration and washed with isopropanol and ethyl acetate, then dried *in vacuo* at ambient temperature for 3 to 4 hours. A solid weighing 0.207 g was obtained. Quantitative analysis versus standard showed it contained 36.2% of the desired product.

Method II

To a solution of 2-aminooxy-2-methylpropanoic acid hydrochloride salt (0.186 kg) in water (1.5 liters) and acetonitrile (1.5 liters), preadjusted to pH 2.0 to 2.2 by addition of triethylamine, was added (3S-*trans*)-3-[[[(2-(formylamino)-4-thiazolyl]oxoacetyl]amino]-4-methyl-2-oxo-1-azetidinesulfonic acid, potassium salt (see example 1A, 0.278 kg) in two equal portions and the suspension was stirred at 20°C for 8 hours while the pH was maintained at pH 2.0-2.2 by further addition of triethylamine. To the resulting clear solution was added concentrated hydrochloric acid (216 ml) with vigorous stirring which was continued for 12 hours at 20°C. The resulting slurry was adjusted to pH 3.5-4.0 by the addition of saturated aqueous potassium bicarbonate and extracted with dichloromethane (3 liters). The aqueous layer was then treated with 3 liters of aqueous tetrabutylammonium hydrogen sulfate (0.7 kg), adjusted to pH 3.5 with saturated aqueous potassium bicarbonate, and the aqueous layer extracted with dichloromethane (6 liters). The dichloromethane layer was dried over sodium sulfate, filtered and treated dropwise with 97% formic acid (345 ml) and stirred at 20°C for 40 minutes. The solids were collected by filtration, washed with methylene chloride and dried *in vacuo* for 4 hours at 50°C and for 12 hours at 25°C to give 0.25 kg of the title compound as a fine white crystalline powder. (Molar yield by quantitation vs standard = 69%).

Example 6

(3S)-3-[[(2-Amino-4-thiazolyl)[(1-carboxyl-1-methylethoxy)imino]acetyl]amino]-2-oxo-1-azetidinesulfonic acid, sodium salt

To a solution of (3S)-3-[[(2-amino-4-thiazolyl)oxoacetyl]amino]-2-oxo-1-azetidinesulfonic acid, potassium salt (90 mg, 0.251 mmol) in water (2 ml) *was* added 2-aminooxy-2-methylpropanoic acid, trifluoroacetate salt (117 mg, 0.503 mmol) and sodium acetate (62 mg, 0.75 mmol). The mixture (pH 4.2) was stirred at room temperature overnight. The pH was then raised to 6.7 with 0.5 N sodium hydroxide, and solvent was removed *in vacuo*. The crude product was chromatographed on HP-20 resin yielding the title compound (52 mg).

**Claims**

1. A process for preparing a compound having the formula

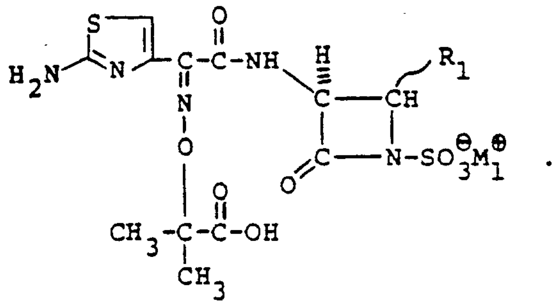

V

which comprises reacting a compound having the formula

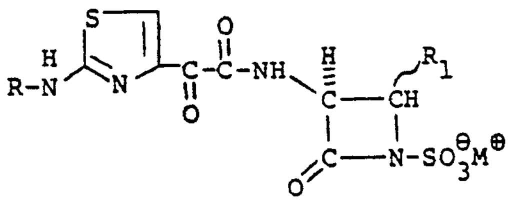

(IV)

obtained by coupling a compound having the formula

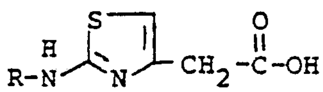

(I)

or a salt thereof, with a compound having the formula

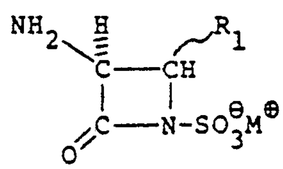

(II)

to yield a compound having the formula

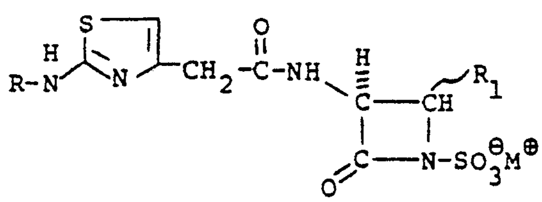

(III)

and oxidizing that compound to yield the reactant of Formula IV with 2-aminooxy-2-methyl-propanoic acid, or a salt thereof and, if R is an amino protecting group, deprotecting that compound to yield the desired product; wherein

R is hydrogen or an amino protecting group;
$R_1$ is hydrogen or $C_1$-$C_4$ alkyl;
$M^{\oplus}$ is an inorganic cation or a substituted ammonium ion; and
$M_1^{\oplus}$ is hydrogen, an inorganic cation or a substituted ammonium ion.

2. A process in accordance with claim 1 wherein R is an amino protecting group.
3. A process in accordance with claim 1 and/or 2 wherein $R_1$ is hydrogen.
4. A process in accordance with claim 1 and/or 2 wherein $R_1$ is α-methyl.
5. A process in accordance with claim 1 wherein R is formyl or hydrogen.

6. A compound having the formula

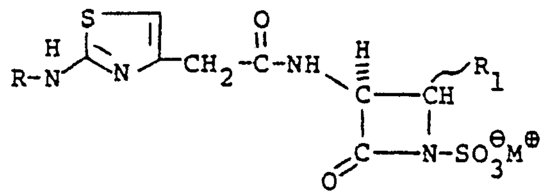

(III)

wherein the symbols are as defined in claim 1.

7. A compound in accordance with claim 6 wherein R is an amino protecting group.

8. A compound in accordance with claim 6 wherein $R_1$ is hydrogen.

9. A compound in accordance with claim 6 wherein $R_1$ is α-methyl.

10. A compound in accordance with claim 6 wherein R is formyl or hydrogen.

**Patentansprüche**

1. Ein Verfahren zur Herstellung einer Verbindung der Formel

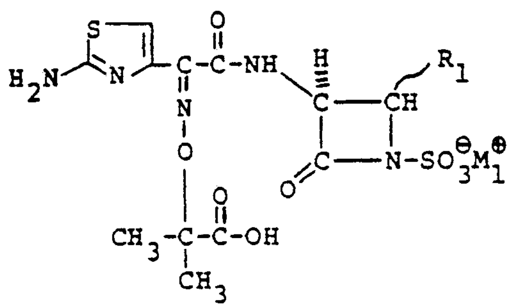

V

welches umfasst die Umsetzung einer Verbindung der Formel

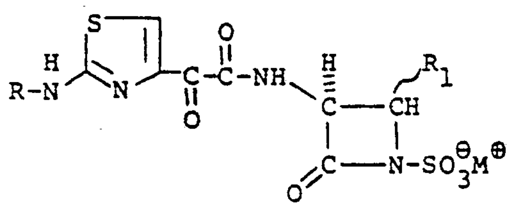

(IV)

erhalten durch Kupplung einer Verbindung der Formel

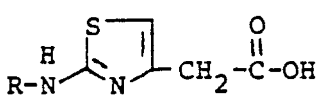

(I)

oder eines Salzes davon mit einer Verbindung der Formel

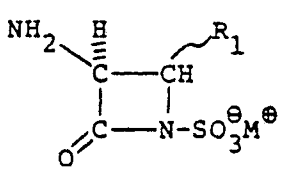

(II)

unter Bildung einer Verbindung der Formel

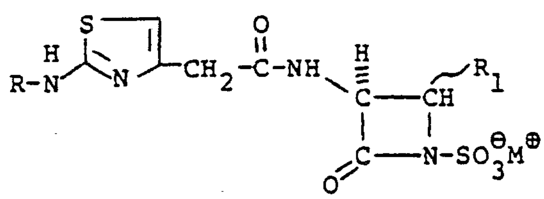

(III)

und Oxidation dieser Verbindung unter Bildung des Reaktanten der Formel IV, mit 2-Aminooxy-2-methylpropanesäure oder einem Salz davon und, wenn R eine Aminoschutzgruppe bedeutet, Entfernen der Schutzgruppe dieser Verbindung unter Bildung des gewünschten Produkts; wobei
R Wasserstoff oder eine Aminoschutzgruppe bedeutet;
$R_1$ Wasserstoff oder $C_1$—$C_4$-Alkyl bedeutet;
$M^{\oplus}$ ein anorganisches Kation oder ein substituiertes Ammoniumion bedeutet; und
$M_1^{\oplus}$ Wasserstoff, ein anorganisches Kation oder ein substituiertes Ammoniumion bedeutet.

2. Ein Verfahren nach Anspruch 1, wobei R eine Aminoschutzgruppe bedeutet.

3. Ein Verfahren nach Anspruch 1 und/oder 2, wobei $R_1$ Wasserstoff bedeutet.

4. Ein Verfahren nach Anspruch 1 und/oder 2, wobei $R_1$ α-Methyl bedeutet.

5. Ein Verfahren nach Anspruch 1, wobei R Formyl oder Wasserstoff bedeutet.

6. Eine Verbindung der Formel

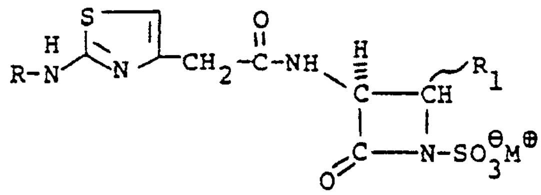

(III)

wobei die Symbole die in Anspruch 1 definierten Bedeutungen haben.

7. Eine Verbindung nach Anspruch 6, wobei R eine Aminoschutzgruppe bedeutet.

8. Eine Verbindung nach Anspruch 6, wobei $R_1$ Wasserstoff bedeutet.

9. Eine Verbindung nach Anspruch 6, wobei $R_1$ α-Methyl bedeutet.

10. Eine Verbindung nach Anspruch 6, wobei R Formyl oder Wasserstoff bedeutet.

**Revendications**

1. Procédé de préparation d'un composé ayant pour formule

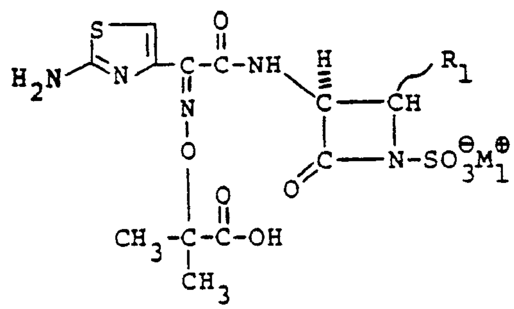

V

qui consiste à faire réagir un composé ayant pour formule

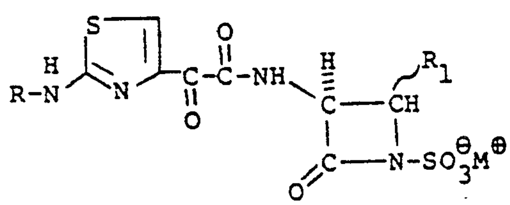

(IV)

obtenu en combinant un composé ayant pour formule

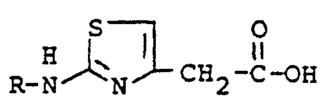

(I)

ou un sel de celui-ci, avec un composé ayant pour formule

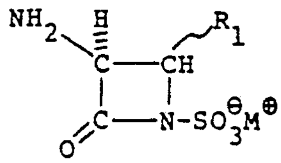

(II)

pour obtenir un composé ayant pour formule

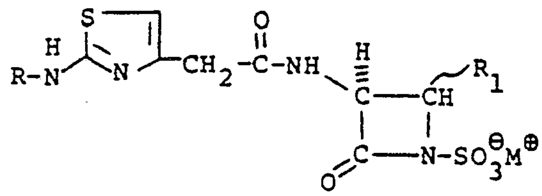

(III)

et à oxyder ce composé pour obtenir le produit réagissant de formule IV avec de l'acide 2-amino-oxy-2-méthyl-propanoïque, ou un sel de celui-ci,

et, si R est un groupe protecteur d'amine, à supprimer la protection de ce composé pour obtenir le produit voulu;

R étant un atome d'hydrogène ou un groupement protecteur d'amine;

$R_1$ étant un atome d'hydrogène ou un radical alkyle en $C_1$ à $C_4$;

$M^+$ étant un cation minéral ou un ion ammonium substitué; et

$M_1^+$ étant un atome d'hydrogène, un cation minéral ou un ion ammonium substitué.

2. Procédé selon la revendication 1, dans lequel R est un groupement protecteur d'amine.

3. Procédé selon la revendication 1 et/ou 2, dans lequel $R_1$ est un atome d'hydrogène.

4. Procédé selon la revendication 1 et/ou 2, dans lequel $R_1$ est un radical α-méthyle.

5. Procédé selon la revendication 1, dans lequel R est un radical formyle ou un atome d'hydrogène.

6. Composé ayant pour formule

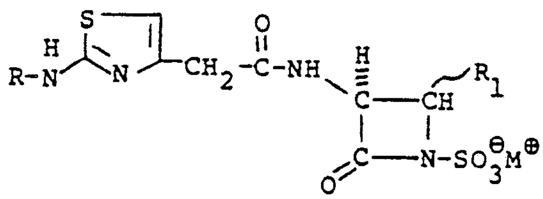

(III)

,

dans laquelle les symboles ont les mêmes définitions que dans la revendication 1.

7. Composé selon la revendication 6, dans lequel R est un groupement protecteur d'amine.

8. Composé selon la revendication 6, dans lequel $R_1$ est un atome d'hydrogène.

9. Composé selon la revendication 6, dans lequel $R_1$ est un radical α-méthyle.

10. Composé selon la revendication 6, dans lequel R est un radical formyle ou un atome d'hydrogène.